(19) Europäisches Patentamt
European Patent Office
Office européen des brevets

(11) Veröffentlichungsnummer : **0 039 029**
**B1**

# (12) EUROPÄISCHE PATENTSCHRIFT

(45) Veröffentlichungstag der Patentschrift : **04.07.84**

(51) Int. Cl.³ : **C 11 B 9/00**, A 61 K 7/46

(21) Anmeldenummer : **81102987.5**

(22) Anmeldetag : **18.04.81**

(54) **Verwendung substituierter 2-(1-Methylbutyl)-1,3-dioxane als Riechstoffe sowie diese enthaltende Riechstoffkompositionen.**

(30) Priorität : **25.04.80 DE 3016007**

(43) Veröffentlichungstag der Anmeldung : **04.11.81 Patentblatt 81/44**

(45) Bekanntmachung des Hinweises auf die Patenterteilung : **04.07.84 Patentblatt 84/27**

(84) Benannte Vertragsstaaten : **AT BE CH DE FR GB IT LI NL**

(56) Entgegenhaltungen :
**FR-A- 2 226 187**
**FR-A- 2 252 814**
**US-A- 3 326 746**
**US-A- 3 423 430**
**COMPTES-RENDUS DE L'ACADEMIE DES SCIENCES, Band 250, 1960, Seiten 3336-3338, Paris, FR. P. MASTAGLI et al.: "Action déshydratante de l'acide molybdique en phase liquide sur les diols"**
**BULLETIN DE LA SOCIETE CHIMIQUE DE FRANCE, Nr. 2, 1969, Seiten 638-650, Paris, FR. T. YVERNAULT et al.: "Sur la déshydratation acide du diméthyl-2,2 propanediol-1,3 et de quelques autres propanediols-1,3 disubstitués en 2. III. Déshydration de quelques bêta-diols aliphatiques "**

(73) Patentinhaber : **Henkel Kommanditgesellschaft auf Aktien**
**Postfach 1100 Henkelstrasse 67**
**D-4000 Düsseldorf-Holthausen (DE)**

(72) Erfinder : **Upadek, Horst, Dr.**
**Kempenweg 18a**
**D-4006 Erkrath 2 (DE)**
Erfinder : **Bruns, Klaus, Dr.**
**Notburgaweg 6**
**D-4150 Krefeld-Traar (DE)**

**EP 0 039 029 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es wurde gefunden, daß substituierte 2-(1-Methylbutyl)-1,3-dioxane der allgemeinen Formel

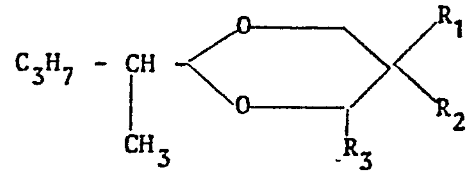

in der $R_1$ Wasserstoff, den Methylrest oder Ethylrest, $R_2$ einen Methyl-, Ethyl-, n-Propyl-, i-Propyl-, n-Butyl- oder sekundären Butylrest, $R_3$ Wasserstoff, den n-Propyl- oder i-Propylrest darstellen, mit der Maßgabe, daß die Summe der Kohlenstoffatome der Reste $R_1$-$R_3 \leq 6$ ist, in vorteilhafter Weise als Riechstoffe mit einer fruchtigen oder blumigen Geruchsnote verwendet werden können.

Die Herstellung der erfindungsgemäß als Riechstoffe zu verwendenden substituierten 2-(1-Methylbutyl)-1,3-dioxane erfolgt nach allgemein bekannten Methoden der organischen Synthese durch Acetalisierung von 2-Methylpentanal mit entsprechenden aliphatischen 1,3-Diolen. Die Reaktion verläuft dabei nach folgendem Schema, wobei die Reste $R_1$-$R_3$ die vorstehende Bedeutung haben :

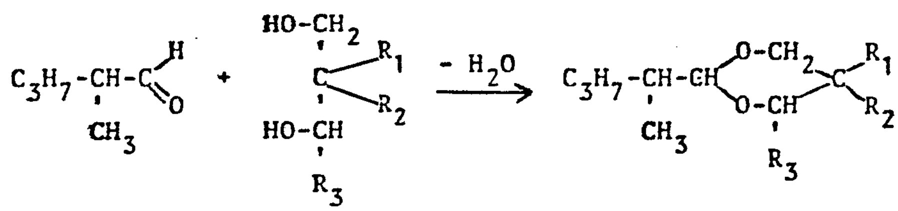


Als erfindungsgemäß zu verwendende substituierte 2-(1-Methylbutyl)-1,3-dioxane sind zum Beispiel 2-(1-Methyl-butyl)-5-methyl-5-n-propyl-1,3-dioxan, -5,5-dimethyl-1,3-dioxan, -5,5-dimethyl-4-n-propyl-1,3-dioxan, -5,5-dimethyl-4-i-propyl-1,3-dioxan, -5-ethyl-4-n-propyl-1,3-dioxan, -5,5-diethyl-1,3-dioxan, -5-methyl-5-sek. butyl-1,3-dioxan, -5-methyl-5-ethyl-1,3-dioxan, -5-ethyl-5-n-butyl-1,3-dioxan zu nennen.

Die erfindungsgemäß zu verwendenden substituierten 2-(1-Methylbutyl)-1,3-dioxane stellen wertvolle Riechstoffe mit charakteristischen fruchtigen und blumigen Geruchsnoten dar. Ihr besonderer Vorteil ist ihre sehr gute Kombinationsfähigkeit zu neuartigen Geruchsnuancen. Besondere Bedeutung kommt dabei dem 2-(1-Methyl-butyl)-5-methyl-5-n-propyl-1,3-dioxan mit einer interessanten Liguster-/Cachounote zu.

Aus verschiedenen Literaturstellen, wie zum Beispiel P. Mastagli und Ch. de Fournas, Compt. Rend. 250 (1960) Seite 3336 bis 3338 und T. Yvernault, M. Mazet, Bull. Soc. Chim. Fr. 1969, Seite 638 sind bereits substituierte 2-(1-Methylbutyl)-1,3-dioxane bekannt geworden. Angaben über Riechstoffeigenschaften oder gar der Hinweis auf eine Verwendungsmöglichkeit als Riechstoffe finden sich dort jedoch nicht.

Die erfindungsgemäß zu verwendenden substituierten 2-(1-Methylbutyl)-1,3-dioxane können mit anderen Riechstoffen in der verschiedensten Mengenverhältnissen zu neuen Riechstoffkompositionen gemischt werden. Im allgemeinen wird sich jedoch der Anteil der erfindungsgemäß zu verwendenden substituierten 2-(1-Methylbutyl)-1,3-dioxane in den Riechstoffkompositionen in den Mengen von 1 bis 50 Gewichtsprozent, bezogen auf die gesamte Komposition, bewegen. Derartige Kompositionen können direkt als Parfum oder auch zur Parfumierung von Kosmetika, wie Cremes, Lotionen, Duftwässern, Aerosolen, Toiletteseifen und so weiter dienen. Sie können aber auch zur Geruchsverbesserung technischer Produkte wie Wasch- und Reinigungsmitteln, Desinfektionsmitteln, Textilbehandlungsmitteln und so weiter eingesetzt werden.

Die nachfolgenden Beispiele sollen den Gegenstand der Erfindung näher erläutern, ohne ihn jedoch hierauf zu beschränken.

## Beispiele

Zunächst wird nachfolgend eine Reihe der erfindungsgemäß zu verwendenden substituierten 2-(1-Methylbutyl)-1,3-dioxane sowie eine allgemeine Arbeitsvorschrift zu ihrer Herstellung näher beschrieben.

### Allgemeine Arbeitsvorschrift

0,1 Mol 2-Methylpentanal, 0,1 Mol 1,3-Alkandiol, 0,1 Mol Orthoameisensäuretriethylester und 0,5 g p-Toluolsulfonsäure wurden 1 Stunde lang bei Raumtemperatur verrührt. Danach wurde langsam ein Gemisch aus Ameisensäureethylester und Ethanol abdestilliert. Der abgekühlte Rückstand wurde in Ether aufgenommen, mit Sodalösung und Wasser gewaschen, über Natriumsulfat getrocknet, eingeengt und im Vakuum destilliert. Nach dieser Arbeitsweise wurden alle nachstehend aufgeführten substituierten 2-(1-Methylbutyl)-1,3-dioxane hergestellt.

1. 2-(1-Methylbutyl)-5-methyl-5-n-propyl-1,3-dioxan
   Sdp. 72 °C/0,02 mbar $n_D^{20}$ 1,4442 1
   Geruch : Liguster-, Cachou-Note

2. 2-(1-Methylbutyl)-5,5-dimethyl-1,3-dioxan
   Sdp. 75 °C/9 mbar $n_D^{20}$ 1,433 7
   Geruch : fruchtig, Johannisbrot-Note

3. 2-(1-Methylbutyl)-5,5-dimethyl-4-n-propyl-1,3-dioxan
   Sdp. 73 °C/1,5 mbar $n_D^{20}$ 1,439 7
   Geruch : fruchtig, Blaubeer-Note

4. 2-(1-Methylbutyl)-5,5-dimethyl-4-i-propyl-1,3-dioxan
   Sdp. 77 °C/0,02 mbar $n_D^{20}$ 1,440 5
   Geruch : fruchtig

5. 2-(1-Methylbutyl)-5-ethyl-4-n-propyl-1,3-dioxan
   Sdp. 82 °C/0,2 mbar $n_D^{20}$ 1,442 2
   Geruch : fruchtig, metallisch, Humus-Note

6. 2-(1-Methylbutyl)-5,5-diethyl-1,3-dioxan
   Sdp. 77 °C/1 mbar $n_D^{20}$ 1,446 9
   Geruch : fruchtig, Vigorose-, Erdbeer-Note

7. 2-(1-Methylbutyl)-5-methyl-5-sek. butyl-1,3-dioxan
   Sdp. 90 °C/0,13 mbar $n_D^{20}$ 1,449 5
   Geruch : metallisch, Geranien-Note

8. 2-(1-Methylbutyl)-5-methyl-5-ethyl-1,3-dioxan
   Sdp. 103 °C/17 mbar $n_D^{20}$ 1,440 4
   Geruch : Citronellol-, Isononylalkohol-Note, Kleie

9. 2-(1-Methylbutyl)-5-ethyl-5-n-butyl-1,3-dioxan
   Sdp. 102 °C/2 mbar $n_D^{20}$ 1,448 8
   Geruch : Costus-Note

Rosen-Komplex

| | |
|---|---|
| 2-(1-Methylbutyl)-5,5-diethyl-1,3-dioxan | 100 Gew.-Teile |
| 2-(1-Methylbutyl)-5-methyl-5-sek. butyl-1,3-dioxan | 60 Gew.-Teile |
| Citronellol | 250 Gew.-Teile |
| Citronellylacetat | 120 Gew.-Teile |
| Phenylethylalkohol | 100 Gew.-Teile |
| Methyljonon | 100 Gew.-Teile |
| Bergamottöl | 90 Gew.-Teile |
| Citronellylformiat | 80 Gew.-Teile |
| Zimtalkohol | 80 Gew.-Teile |
| Zitronenöl | 5 Gew.-Teile |
| Hydroxycitronellal | 5 Gew.-Teile |
| Phenylacetaldehyddimethylacetal | 5 Gew.-Teile |
| Nonanol | 4 Gew.-Teile |
| Nonanal | 1 Gew.-Teile |

**Ansprüche**

1. Verwendung von substituierten 2-(1-Methylbutyl)-1,3-dioxanen der allgemeinen Formel

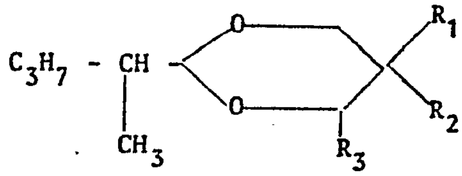

in der $R_1$ Wasserstoff, den Methylrest oder Ethylrest, $R_2$ einen Methyl-, Ethyl, n-Propyl-, i-Propyl-, n-Butyl- oder sekundären Butylrest, $R_3$ Wasserstoff, den n-Propyl- oder i-Propylrest darstellen, mit der Maßgabe, daß die Summe der Kohlenstoffatome der Reste $R_1$-$R_3 \leq 6$ ist, als Riechstoffe.

2. Kompositionen mit wenigstens zwei Riechstoffen, gekennzeichnet durch einen Gehalt an substituierten 2-(1-Methylbutyl)-1,3-dioxanen nach Anspruch 1.

3. Riechstoffkompositionen nach Anspruch 2, dadurch gekennzeichnet, daß sie die substituierten 2-(1-Methyl-butyl)-1,3-dioxane in einer Menge von 1 bis 50 Gewichtsprozent auf die gesamte Komposition, enthalten.

**Claims**

1. The use of substituted 2-(1-methylbutyl)-1,3-dioxanes corresponding to the following general formula

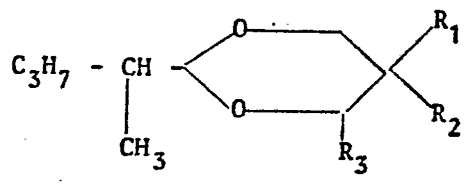

in which $R_1$ represents hydrogen, the methyl radical or ethyl radical, $R_2$ represents a methyl, ethyl, n-propyl, i-propyl, n-butyl or secondary butyl radical, $R_3$ represents hydrogen, the n-propyl or i-propyl radical, with the proviso that the sum of the carbon atoms of the radicals $R_1$ to $R_3 \leq 6$, as perfumes.

2. Compositions containing at least two perfumes, characterized by a content of substituted 2-(1-methylbutyl)-1,3-dioxanes corresponding to Claim 1.

3. Perfume compositions as claimed in Claim 2, characterized in that they contain the substituted 2-(1-methylbutyl)-1,3-dioxanes in a quantity of from 1 to 50 % by weight, based on the composition as a whole.

**Revendications**

1. Utilisation de 2-(1-méthylbutyl)-1,3-dioxannes substitués de formule générale

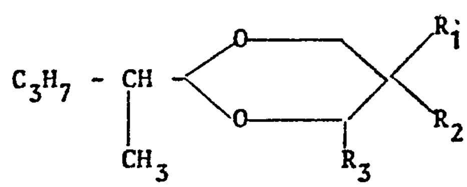

dans laquelle $R_1$ représente de l'hydrogène, le radical méthyle ou le radical éthyle, $R_2$ un radical méthyle, éthyle, n-propyle, isopropyle, n-butyle ou butyle secondaire, $R_3$ de l'hydrogène, le radical n-propyle ou le radical isopropyle, avec comme condition que la somme des atomes de carbone des radicaux $R_1$ à $R_3$ soit égale ou inférieure à 6, en tant que matières odorantes.

2. Compositions avec au moins deux matières odorantes, caractérisées par une teneur en 2-(1-méthylbutyl)-1,3-dioxannes substitués selon la revendication 1.

3. Compositions de matières odorantes selon la revendication 2, caractérisées en ce qu'elles contiennent les 2-(1-méthylbutyl)-1,3-dioxannes substitués en une quantité de 1 à 50 % en poids par rapport à la composition totale.